# EUROPEAN PATENT APPLICATION

(11) **EP 4 537 841 A1**
(43) Date of publication of application: **16.04.2025**
(21) Application number: 23819825.3
(22) Date of filing: 06.06.2023
(51) Int. Cl.: A61K 39/395, A61P 3/10, A61P 13/12, A61P 43/00, C07K 16/18

(54) **PHARMACEUTICAL COMPOSITION CONTAINING ANTI-INTEGRIN ALPHA-11 ANTIBODY FOR TREATMENT OR PREVENTION OF AGING-RELATED DISEASES**

(30) Priority: 07.06.2022 JP 2022092195
(71) Applicant: Astellas Pharma, Inc., Chuo-ku Tokyo 103-8411 (JP)
(72) Inventor: UCHINO, Hiroshi, Tokyo 103-8411 (JP)
(74) Representative: Hoffmann Eitle
(86) International application number: PCT/JP2023/020932
(87) International publication number: WO 2023/238845

(57) **Abstract**

Provided is a novel pharmaceutical composition for treating or preventing an aging-related disease by utilizing selective removal of a senescent cell. A pharmaceutical composition comprising an anti-integrin α11 (ITGA11) antibody as an active ingredient is administered to a subject.

## Description

### TECHNICAL FIELD

The present invention relates to a pharmaceutical composition comprising an anti-integrin α11 (ITGA11) antibody for treating or preventing an aging-related disease, a method for selectively removing a senescent cell, a method for treating or preventing an aging-related disease, and others.

### BACKGROUND ART

Cellular senescence refers to the state of a cell defined by permanent cessation of cell division and generally seen when cell division reaches the upper limit or when DNA is damaged by stress or activation of a cancer gene. A cell senesced (hereinafter, referred to as "senescent cell") highly expresses p16 protein and p21 protein and is characterized by an increase level of SAHF (Senescence-associated Heterochromatin Foci). In addition, the senescent cell releases SASP (Senescence-associated Secretory Phenotype) factors causing inflammation. Examples of the SASP factors comprise interleukins (IL-1a, IL-1b, IL-6, etc.), chemokines (IL-8, CXCL1, etc.), growth factors (bEGF, HGF, etc.), and proteases (MMP-1, MMP-3, MMP-13, etc.). It is considered that SASP factors released from a senescent cell have adverse effects on peripheral cells, leading to exacerbation of disease conditions.

As the diseases that can be treated by removing such a senescent cell, idiopathic pulmonary fibrosis, hepatic fibrosis, sarcopenia, type 2 diabetes, and cataract have been reported (Nat. Rev. Mol. Cell Biol., Vol. 15, page 482-496 (2014)).

Attempts to identify a surface protein for distinguishing cellular senescence have been made by Kim, et al., and DPP4 and REEP5 were reported (NPL 1). Actually, it has been confirmed that DPP4 increases at an mRNA level and a protein level with an aging stimulus given by the passage of a cell. Furthermore, it has been reported that DPP4 present in the cell surface can be recognized by an anti-DPP4 antibody. Moreover, it has been reported that if an NK cell is activated via an anti-DPP4 antibody, antibody-dependent cytotoxicity can be induced to remove a senescent cell.

However, DPP4 is expressed in various normal tissues including intestinal cells. Since the expression of DPP4 is not specific to a senescent cell, it is difficult to remove a senescent cell *in vivo* by an anti-DPP4 antibody in a senescent cell-specific manner. Accordingly, to more selectively remove a senescent cell, it is necessary to identify a protein specifically expressed on a cell surface with cell aging.

Examples of an antibody to human ITGA11 that have been reported comprise mouse monoclonal antibody A03 (PTL 1), human monoclonal antibody NB0268 (PTL 2), chicken-mouse chimeric monoclonal antibody #33-46 (PTL 3), and human monoclonal antibody 210F4B6A4 (PTL 4). In addition, it has been reported that human monoclonal antibody NB0268 (PTL 2) and chicken-mouse chimeric monoclonal antibody #33-46 (PTL 3) have a cell-adhesion inhibitory activity.

However, it has not been reported that a senescent cell is removed by a human ITGA11 antibody to treat an aging-related disease.

### CITATION LIST

### PATENT LITERATURE

PTL 1: International Publication No. WO2008/075045
PTL 2: Canadian Patent Application Publication No. 2985274
PTL 3: International Publication No. WO2019/168176
PTL 4: European Patent Application Publication No. 3517549

### NON PATENT LITERATURE

NPL 1: Genes Dev., Vol. 31, page 1529-1534 (2017)

### SUMMARY OF INVENTION

### TECHNICAL PROBLEM

The problem to be solved by the present invention is to provide a novel pharmaceutical composition comprising an anti-ITGA11 antibody for treating or preventing an aging-related disease.

### SOLUTION TO PROBLEM

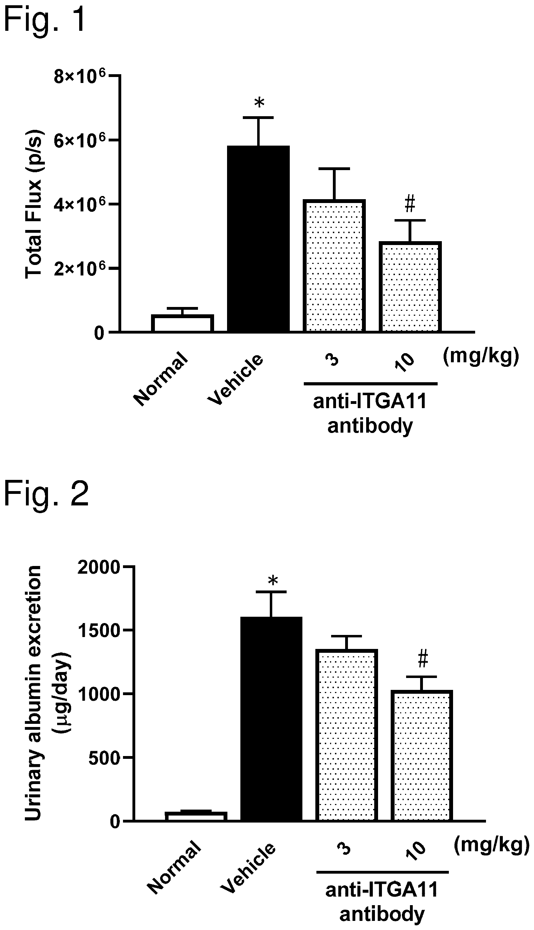

The present inventors conducted intensive studies with a view to solving the above problem. As a result, they found that if an anti-ITGA11 antibody is administered to a subject, the activity of antibody-dependent cell-mediated cytotoxicity (ADCC) is induced only in a senescent cell. Also, they found that a senescent cell can be removed by administering an anti-ITGA11 antibody *in-vivo* (Fig. 1), and consequently, an aging-related disease can be treated or prevented (Fig. 2). Based on the findings, the present invention was accomplished.

More specifically, the present invention relates to a pharmaceutical composition comprising an anti-ITGA11 antibody for treating or preventing an aging-related disease and others. In an embodiment, the present invention is as follows.
[1] A pharmaceutical composition for treating or preventing an aging-related disease, wherein the composition comprises an anti-integrin α11 (ITGA11) antibody.
[2] The pharmaceutical composition according to [1], wherein the anti-ITGA11 antibody is comprised in an effective amount for selectively removing a senescent cell.
[3] The pharmaceutical composition according to [1] or [2], wherein the senescent cell is a p16-expressing cell.
[4] The pharmaceutical composition according to any one of [1] to [3], wherein the aging-related disease is selected from the group consisting of:
   (i) atherosclerosis, angina pectoris, arrhythmia, cardiomyopathy, congestive heart failure, coronary artery disease, carotid artery disease, endocarditis, coronary artery thrombosis, myocardial infarction, hypertension, aortic aneurysm, diastolic dysfunction of the heart, hypercholesterolemia, hyperlipidemia, mitral valve prolapse, peripheral vascular disease, stress tolerance of the heart, myocardial fibrosis, cerebral aneurysm, and stroke;
   (ii) osteoarthritis, osteoporosis, oral mucositis, inflammatory bowel disease, kyphosis, and herniated disc;
   (iii) Alzheimer's disease, Parkinson's disease, Huntington's disease, dementia, mild cognitive impairment, and motor neuron dysfunction;
   (iv) diabetes, diabetic ulcer, metabolic syndrome, and obesity;
   (v) pulmonary fibrosis, chronic obstructive pulmonary disease, asthma, cystic fibrosis, emphysema, bronchiectasis, and decreased pulmonary function;
   (vi) macular degeneration, glaucoma, cataract, presbyopia, decreased vision, and loss of vision; and
   (vii) kidney disease, kidney failure, diabetic nephropathy, weakness, hearing loss, muscle fatigue, skin disease, skin wound healing, hepatic fibrosis, pancreatic fibrosis, oral submucosal tissue fibrosis, dry eye and sarcopenia;
   (viii) residual cancer after chemotherapy, residual cancer after radiotherapy and atrophy or dysfunction of the peripheral normal tissue, dysfunction of tissue transplant associated with ischemia-reperfusion (kidney transplant, heart transplant, liver transplant);
   (ix) response failure to vaccine.
[5] The pharmaceutical composition according to any one of [1] to [4], wherein the ITGA11 antibody comprises a heavy chain consisting of the amino acid sequence represented by SEQ ID NO: 2 and a light chain consisting of the amino acid sequence represented by SEQ ID NO: 4.
[6] The pharmaceutical composition according to any one of [1] to [5], wherein the ITGA11 antibody comprises a heavy chain consisting of an amino acid sequence encoded by a nucleotide sequence represented by SEQ ID NO: 1 and a light chain consisting of the amino acid sequence encoded by a nucleotide sequence represented by SEQ ID NO: 3.
[7] The pharmaceutical composition according to any one of [1] to [6], further comprising a pharmaceutically acceptable carrier or additive.
[8] A method for treating or preventing an aging-related disease, including administering an effective amount of an anti-ITGA11 antibody for selectively removing a senescent cell, to a subject.
[9] The method according to [8], wherein the senescent cell is a p16-expressing cell.
[10] The method according to [8] or [9], where the aging-related disease is selected from the group consisting of:
   (i) atherosclerosis, angina pectoris, arrhythmia, cardiomyopathy, congestive heart failure, coronary artery disease, carotid artery disease, endocarditis, coronary artery thrombosis, myocardial infarction, hypertension, aortic aneurysm, diastolic dysfunction of the heart, hypercholesterolemia, hyperlipidemia, mitral valve prolapse, peripheral vascular disease, stress tolerance of the heart, myocardial fibrosis, cerebral aneurysm, and stroke;
   (ii) osteoarthritis, osteoporosis, oral mucositis, inflammatory bowel disease, kyphosis, and herniated disc;
   (iii) Alzheimer's disease, Parkinson's disease, Huntington's disease, dementia, mild cognitive impairment, and motor neuron dysfunction;
   (iv) diabetes, diabetic ulcer, metabolic syndrome, and obesity;
   (v) pulmonary fibrosis, chronic obstructive pulmonary disease, asthma, cystic fibrosis, emphysema, bronchiectasis, and decreased pulmonary function;
   (vi) macular degeneration, glaucoma, cataract, presbyopia, decreased vision, and loss of vision; and
   (vii) kidney disease, kidney failure, diabetic nephropathy, weakness, hearing loss, muscle fatigue, skin disease, skin wound healing, hepatic fibrosis, pancreatic fibrosis, oral submucosal tissue fibrosis, dry eye and sarcopenia;
   (viii) residual cancer after chemotherapy, residual cancer after radiotherapy and atrophy or dysfunction of the peripheral normal tissue, dysfunction of tissue transplant associated with ischemia-reperfusion (kidney transplant, heart transplant, liver transplant);
   (ix) response failure to vaccine.
[11] The method according to any one of [8] to [10], wherein the ITGA11 antibody comprises a heavy chain consisting of an amino acid sequence represented by SEQ ID NO: 2 and a light chain consisting of an amino acid sequence represented by SEQ ID NO: 4.
[12] The method according to any one of [8] to [11], wherein the ITGA11 antibody comprises a heavy chain consisting of an amino acid sequence encoded by a nucleotide sequence represented by SEQ ID NO: 1 and a light chain consisting of an amino acid sequence encoded by the nucleotide sequence represented by SEQ ID NO: 3.
[13] An anti-ITGA11 antibody for use in treating or preventing an aging-related disease.
[14] Use of an anti-ITGA11 antibody for treating or preventing an aging-related disease.
[15] Use of an anti-ITGA11 antibody for producing a pharmaceutical composition for treating or preventing an aging-related disease.

### ADVANTAGEOUS EFFECTS OF INVENTION

The present invention can be used for treating or preventing an aging-related disease, since administering, e.g., a pharmaceutical composition comprising an anti-ITGA11 antibody to a subject can selectively remove a senescent cell.

### BRIEF DESCRIPTION OF DRAWINGS

[Fig. 1] Fig. 1 shows the measurement results of the expression levels of p16 serving as an indicator for a senescent cell in the kidney, 3 weeks after anti-ITGA11 antibody #014 was administered to type 1 (insulin-dependent) diabetic model mice prepared by intraperitoneally administering a streptozotocin (STZ) solution (200 mg/kg) to p16-luc mice (52 weeks old). "Normal" represents p16-luc mice not receiving STZ; "Vehicle" represents STZ-induced diabetes model p16-luc mice receiving administration of solvent D-PBS; and "Anti-ITGA11 antibody" represents a group of STZ-induced diabetes model p16-luc mice receiving administration of anti-ITGA11 antibody #014 at a dose of 3 mg/kg or 10 mg/kg once a week continuously for 3 weeks. The vertical axis indicates the luminescence of luciferase (Total Flux (p/s)) in the kidney. Data are shown by mean value + standard error (n = 7 or 8); Student's t-test and Dunnett's test were used as the statistical test; and the significance level was set as p < 0.05 (*: Student's t-test p < 0.05 vs Normal group; #: Dunnett's test p < 0.05 vs Vehicle group).

[Fig. 2] Fig. 2 shows the measurement results of urinary albumin secretion used as evaluation of kidney function 3 weeks after anti-ITGA11 antibody #014 was administered to type-1 (insulin-dependent) diabetic model mice prepared by intraperitoneally administering a streptozotocin (STZ) solution (200 mg/kg) to p16-luc mice (52 weeks old). "Normal" represents p16-luc mice not receiving STZ; "Vehicle" represents STZ-induced diabetes model p16-luc mice receiving administration of solvent D-PBS; and "Anti-ITGA11 antibody" represents a group of STZ-induced diabetes model p16-luc mice receiving administration of anti-ITGA11 antibody #014 at a dose of 3 mg/kg or 10 mg/kg once a week continuously for 3 weeks. The vertical axis indicates urinary albumin secretion (µg/day). The data are shown by a mean value + standard error (n = 8); Student's t-test and Dunnett's test were used as the statistical test; the significant level was p < 0.05 (*: Student's t-test p < 0.05 vs Normal group; #: Dunnett's test p < 0.05 vs Vehicle group).

### DESCRIPTION OF EMBODIMENTS

The present invention will be more specifically described below, but the present invention is not limited to these descriptions. In the specification, unless otherwise specified, scientific terms and technical terms used in connection with the present invention have meanings commonly understood by those skilled in the art.

### <Pharmaceutical Composition>

In an embodiment, the present invention relates to a pharmaceutical composition comprising an anti-ITGA11 antibody for treating or preventing an aging-related disease and others. The basic structures of antibody molecules are common in all classes. The antibody is constituted of heavy chains having a molecular weight of 50,000 to 70,000 and light chains having a molecular weight of 20,000 to 30,000. The heavy chain is constituted of a polypeptide chain usually comprising about 440 amino acids and has a characteristic structure depending on the class. The heavy chains of IgG, IgM, IgA, IgD, and IgE are called as Igγ, Igµ, Igα, Igδ, and Igε chains, respectively. IgG further has subclasses such as IgG1, IgG2, IgG3, and IgG4 and called Igy1, Igy2, Igy3, and Igy4, respectively. The light chain is constituted of a polypeptide chain usually comprising about 220 amino acids. Two types of light chains, λ type and κ type, are known and called Igλ and Igκ, respectively. A basic structure (peptide structure) of the antibody molecule consists of two homologous heavy chains and two homologous light chains which are bound via a disulfide bond (S-S bond) and a non-covalent bond. The molecular weight is 150,000 to 190,000. The two types of light chains can form a pair with either one of the heavy chains. Individual antibody molecules are each principally formed of identical two light chains and identical two heavy chains.

The number of the intrachain S-S bonds present in a heavy chain is 4 (5 in µ and ε chains) and 2 in a light chain. A loop is formed every 100 to 110 amino acid residues. The three-dimensional structures of the loops are analogous and called structural units or domains. The domains positioned at the N terminals of both heavy chain and light chain, even if derived from the same class (subclass) of the same species, vary in amino acid sequence and are called variable regions. These domains are called a heavy-chain variable region (VH) and a light-chain variable region (VL), respectively. The amino acid sequences closer to the C terminal than the variable regions are almost the same within classes or subclasses and called constant regions. The domains thereof are called CH1, CH2, CH3 or CL.

The antigen determination site of an antibody is constituted of VH and VL and the specificity of the binding is determined depending on the amino acid sequence of the site. In contrast, biological activities such as bindings to a complement and various cells reflect the differences in the structure of the constant regions of Ig classes. It is known that the variability of variable regions of a heavy chain and a light chain is almost limited to 3 small hypervariable regions present in either one of the chains. These regions are called complementarity determining regions (CDRs; individually called CDR1, CDR2, CDR3 in the order from the N terminal side). The remaining region of the variable region is called a framework region (FR), which is relatively constant.

In the present invention, the "anti-ITGA11 antibody" may be an antigen-binding fragment in an embodiment. The region between CH1 domain and CH2 domain of a heavy-chain constant region of an antibody is called a hinge region. The hinge region highly comprises a proline residue and a plurality of interchain S-S bonds connecting two heavy chains. For example, the hinge regions of human IgG1, IgG2, IgG3 and IgG4 comprise, 2, 4, 11, and 2 cysteine residues, respectively, which constitute an S-S bond between the heavy chains. The hinge region is a region highly sensitive to a protein-degrading enzyme such as papain and pepsin. When an antibody is digested with papain, the heavy chain is cleaved at a position of the hinge region closer to the N terminal than the inter-heavy chain S-S bond to be broken down into two Fab fragments and a single Fc fragment. The Fab fragment is constituted of a light chain and a heavy-chain fragment comprising a heavy-chain variable region, CH1 domain and a part of the hinge region. When an antibody is digested with pepsin, the heavy chain is cleaved at a portion of the hinge region closer to the C terminal than the inter-heavy chain S-S bond to generate a F (ab')₂ fragment. The F (ab')₂ fragment is a fragment having a dimer structure formed of 2 Fab' fragments bound via the inter-heavy chain S-S bond in the hinge region. The Fab' fragment is constituted of a light chain and a heavy chain fragment comprising a heavy-chain variable region, CH1 domain and part of the hinge region. The hinge region comprises a cysteine residue that previously constituted an inter-heavy chain S-S bond. The Fab fragment, F (ab')₂ fragment and Fab' fragment all comprise a variable region and have antigen binding activity. The anti-ITGA11 antibody to be comprised in the pharmaceutical composition of the present invention is a Fab fragment, a F (ab')₂ fragment, or a Fab' fragment of the anti-ITGA11 antibody in an embodiment.

In an embodiment, the anti-ITGA11 antibody to be comprised in the pharmaceutical composition of the present invention is an anti-ITGA11 antibody comprising the heavy chain constituted of the amino acid sequence represented by SEQ ID NO: 2 and the light chain constituted of the amino acid sequence represented by SEQ ID NO: 4.

In an embodiment, the anti-ITGA11 antibody to be comprised in the pharmaceutical composition of the present invention is an anti-ITGA11 antibody comprising a heavy chain constituted of the amino acid sequence encoded by the nucleotide sequence represented by SEQ ID NO: 1 and a light chain consisting of an amino acid sequence encoded by the nucleotide sequence represented by SEQ ID NO: 3.

Examples of the anti-ITGA11 antibody to be comprised in the pharmaceutical composition of the present invention comprise antibody #014 that will be described later in Examples. Antibody #014 comprises a heavy-chain variable region and a light-chain variable region of NB0314, which is described as a modified antibody improved in the physical property of NB0268 in PTL 2. The heavy-chain variable region of NB0314 is constituted of the amino acid sequence of No. 1 to 120 amino acids of SEQ ID NO: 2 and comprises CDR1 consisting of the amino acid sequence of No. 26 to 35 amino acids of SEQ ID NO: 2, CDR2 consisting of the amino acid sequence of No. 50 to 66 amino acids of SEQ ID NO: 2, and CDR3 consisting of the amino acid sequence of No. 99 to 109 amino acids of SEQ ID NO: 2. The light-chain variable region of NB0314 is constituted of the amino acid sequence of No. 1 to 110 amino acids of SEQ ID NO: 4 and comprises CDR1 consisting of the amino acid sequence of No. 29 to 34 amino acids of SEQ ID NO: 4, CDR2 consisting of the amino acid sequence constituted of No. 50 to 55 amino acids of SEQ ID NO: 4, and CDR3 consisting of the amino acid sequence of No. 89 to 99 amino acids of SEQ ID NO: 4 (above descriptions all in PTL 2).

In an embodiment, the anti-ITGA11 antibody to be comprised in the pharmaceutical composition of the present invention is an anti-ITGA11 antibody comprising a heavy-chain variable region comprising CDR1 consisting of the amino acid sequence of No. 26 to 35 amino acids of SEQ ID NO: 2, CDR2 consisting of the amino acid sequence of No. 50 to 66 amino acids of SEQ ID NO: 2, and CDR3 consisting of the amino acid sequence of No. 99 to 109 amino acids of SEQ ID NO: 2 and a light-chain variable region comprising CDR1 consisting of the amino acid sequence constituted of No. 29 to 34 amino acids of SEQ ID NO: 4, CDR2 consisting of the amino acid sequence constituted of No. 50 to 55 amino acids of SEQ ID NO: 4, and CDR3 consisting of the amino acid sequence constituted of No. 89 to 99 amino acids of SEQ ID NO: 4.

In an embodiment, the anti-ITGA11 antibody to be comprised in the pharmaceutical composition of the present invention is an anti-ITGA11 antibody comprising a heavy-chain variable region consisting of the amino acid sequence of No. 1 to 120 amino acids of SEQ ID NO: 2 and a light-chain variable region consisting of the amino acid sequence of No. 1 to 110 amino acids of SEQ ID NO: 4.

The anti-ITGA11 antibody to be comprised in the pharmaceutical composition of the present invention can be easily prepared by those skilled in the art using a known method in the field to which the invention pertains based on the sequence information disclosed in the specification. For example, a polynucleotide comprising a nucleotide sequence encoding a heavy chain of the anti-ITGA11 antibody and a polynucleotide comprising a nucleotide sequence encoding a light chain thereof are synthesized, and ligated to an appropriate expression vector. Then, the expression vector is introduced in a cultured cell. Finally, the cultured cell is cultured. From the culture supernatant, a monoclonal antibody can be obtained. Such a synthesis method for a polynucleotide, introduction of the polynucleotide into an expression vector, introduction of an expression vector into a cultured cell, culture of the cultured cell and purification of an antibody can be performed using various methods known in the field to which the invention pertains, for example, a method described in PTL 2.

It is known that when an antibody is expressed in a cell, the antibody is post-translationally modified. Examples of the post-translational modification comprise cleavage of lysine at the heavy-chain C-terminal with carboxypeptidase, modification of glutamine or glutamic acid at the heavy chain and light chain N terminals into pyroglutamic acid by pyroglutamylation, glycosylation, oxidation, deamidation, and glycation. Such a post-translational modification is known to occur in various antibodies (J. Pharm. Sci., Vol. 97, page 2426-2447 (2008)).

The anti-ITGA11 antibody to be comprised in the pharmaceutical composition of the present invention may also comprise an anti-ITGA11 antibody generated by post-translational modification. Examples of anti-ITGA11 antibodies that can be generated by post-translational modification comprise an anti-ITGA11 antibody having a heavy-chain variable region with the N terminal pyroglutamylated. It is known in the field to which the invention pertains that such post-translational modification of the N terminal by pyroglutamylation does not affect the activity of the antibody (Anal. Biochem., Vol. 348, page 24-39 (2006)).

The anti-ITGA11 antibody to be comprised in the pharmaceutical composition of the present invention binds to ITGA11. Also, the anti-ITGA11 antibody to be comprised in the pharmaceutical composition of the present invention exhibits ADCC activity in a senescent cell-specific manner. As a method for measuring the binding activity of an anti-ITGA11 antibody to ITGA11, and ADCC activity thereof, various methods known in the field to which the invention pertains can be used.

The present invention relates to a pharmaceutical composition comprising an anti-ITGA11 antibody for treating or preventing an aging-related disease, as mentioned above. An aging-related disease can be treated and prevented by selectively removing a senescent cell generated with aging. Thus, in an embodiment, the present invention relates to a pharmaceutical composition for treating or preventing an aging-related disease, comprising an effective amount of an anti-ITGA11 antibody for selectively removing a senescent cell.

In the specification, the "senescent cell" refers to a cell that reaches the upper limit of cell division, a cell having DNA damaged by stress or activation of a cancer gene, and a cell that ceased to divide. These senescent cells highly express p16 protein and p21 protein. Thus, in an embodiment, the senescent cell of the present invention may be a p16-expressing cell and a p21-expressing cell.

In the specification, examples of the "aging-related disease" comprise, but are not particularly limited to, (i) atherosclerosis, angina pectoris, arrhythmia, cardiomyopathy, congestive heart failure, coronary artery disease, carotid artery disease, endocarditis, coronary artery thrombosis, myocardial infarction, hypertension, aortic aneurysm, diastolic dysfunction of the heart, hypercholesterolemia, hyperlipidemia, mitral valve prolapse, peripheral vascular disease, stress tolerance of the heart, myocardial fibrosis, cerebral aneurysm, and stroke; (ii) osteoarthritis, osteoporosis, oral mucositis, inflammatory bowel disease, kyphosis, and herniated disc; (iii) Alzheimer's disease, Parkinson's disease, Huntington's disease, dementia, mild cognitive impairment, and motor neuron dysfunction; (iv) diabetes, diabetic ulcer, metabolic syndrome, and obesity; (v) pulmonary fibrosis, chronic obstructive pulmonary disease, asthma, cystic fibrosis, emphysema, bronchiectasis, and decreased pulmonary function; (vi) macular degeneration, glaucoma, cataract, presbyopia, decreased vision, and loss of vision; (vii) kidney disease, kidney failure, diabetic nephropathy, weakness, hearing loss, muscle fatigue, skin disease, skin wound healing, hepatic fibrosis, pancreatic fibrosis, oral submucosal tissue fibrosis, dry eye and sarcopenia; (viii) residual cancer after chemotherapy, residual cancer after radiotherapy and atrophy or dysfunction of the peripheral normal tissue, dysfunction of tissue transplant associated with ischemia-reperfusion (kidney transplant, heart transplant, liver transplant); and (ix) response failure to vaccine. In an embodiment, the "aging-related disease" in the present invention is a kidney disease, a kidney failure, or diabetic nephropathy.

In the specification, "treating or preventing an aging-related disease" is, for example, preventing the onset of an aging-related disease by administering the pharmaceutical composition of the present invention before the onset of the aging-related disease or treating an aging-related disease by administering the pharmaceutical composition of the present invention after the onset of the aging-related disease.

In the specification, "selectively remove" means that an antigen-expressing cell is specifically removed via an antigen-specific antibody. The "selectively remove" refers to the removal of an antigen-expressing cell via antibody-dependent phagocytosis in an embodiment, the removal of an antigen-expressing cell via antibody-dependent cytotoxic action in another embodiment, and the removal of an antigen-expressing cell via complement-dependent cytotoxicity in a still another embodiment.

The pharmaceutical composition of the present invention comprises a pharmaceutically acceptable carrier and an additive. The type of pharmaceutically acceptable carrier and the type of additive are not particularly limited and a carrier and additive well known to those skilled in the art can be used. Examples of the carrier to be comprised in the pharmaceutical composition of the present invention comprise distilled water for injection and physiological saline. Examples of the additive to be comprised in the pharmaceutical composition of the present invention comprise a tonicity agent, a buffering agent, a preservative, a wetting agent, an emulsifier, a dispersant, a stabilizer, and a solubilizer.

The pharmaceutical composition of the present invention can be sterilized, for example, by filter filtration, high-pressure steam treatment, dry heat treatment, ethylene oxide gas treatment, UV irradiation, exposure to radiation or addition of a bactericide. Also, the pharmaceutical composition of the present invention is produced as an aseptic solid composition and the solid composition can be dissolved or suspended in aseptic water or an aseptic solvent for injection before use and then put in use.

Examples of the formulation of the pharmaceutical composition of the present invention comprise a parenteral preparation such as an injection, a drip preparation and a depot preparation. Also, the pharmaceutical composition of the present invention may be an aseptic aqueous or non-aqueous solution, a suspension or an emulsion. Examples of the aqueous solvent comprise distilled water for injection and saline solution. Examples of the non-aqueous solvent comprise alcohols such as ethanol.

Examples of the dosage form of the pharmaceutical composition of the present invention comprise, but are not particularly limited to, a local injection to a target tissue such as intra-adipose tissue injection, intramuscular injection, subcutaneous injection, or intravenous injection. The effective amount of the anti-ITGA11 antibody to be comprised in the pharmaceutical composition of the present invention varies depending on the degree of a symptom and the age of a patient, formulation and dosage form of a preparation to be used, or titer of the antibody, and can be, for example, about 0.001 mg/kg to 100 mg/kg.

### <Method for Selectively Removing Senescent Cell and Method for Treating or Preventing Aging-Related Disease>

In an embodiment, the present invention also relates to a method for treating or preventing an aging-related disease, including administering an effective amount of an anti-ITGA11 antibody for selectively removing a senescent cell to a subject. The type of anti-ITGA11 antibody, the amino acid sequence of the antibody and the nucleotide sequence encoding the amino acid sequence, a senescent cell and an aging-related disease are the same as described in the previous section "Pharmaceutical Composition". The "subject" refers to a human or a mammal except a human that requires treating or preventing an aging-related disease. In an embodiment, the "subject" refers to a human who requires treating or preventing an aging-related disease.

The effective amount of an anti-ITGA11 antibody in the method may be the same as the effective amount of an anti-ITGA11 antibody described in the previous section "Pharmaceutical Composition". Also, the dosage form of the anti-ITGA11 antibody in the method is not particularly limited, and for example, local injection to a target tissue such as intra-adipose tissue injection, intramuscular injection, subcutaneous injection, or intravenous injection can be used. The dosage of the anti-ITGA11 antibody in the method varies depending on the degree of a symptom and the age of a subject, formulation and dosage form of a preparation to be used, or titer of the antibody, and is, for example, about 0.001 mg/kg to 100 mg/kg can be used.

In an embodiment, the present invention also relates to an anti-ITGA11 antibody for use in treating or preventing an aging-related disease. In an embodiment, the present invention also relates to use of an anti-ITGA11 antibody for treating or preventing an aging-related disease. In an embodiment, the present invention also relates to use of an anti-ITGA11 antibody for producing a pharmaceutical composition for treating or preventing an aging-related disease.

The present invention has been generally described above. To further facilitate understanding of the invention, specific Examples will be provided hereafter for reference. However, these Examples are provided just for exemplification and do not limit the present invention.

### EXAMPLES

Experiments using, e.g., a commercially available kit or a reagent, were performed in accordance with the protocol attached to the kit or reagent unless otherwise specified.

### Example 1: Preparation of IMR-90 Senescent Cell

IMR-90 cells (ATCC: CCL-186) were seeded in a 96-well cell-culture plate at a density of 1000 cells/well using 200 µL of Eagle's Minimum Essential Medium (Thermo Scientific, 12561-049) comprising 10% bovine serum (HyClone, SH30070.03). After seeding, culture was performed at 37°C in a CO₂ incubator until confluent. The cells were continuously cultured for further 21 days without exchanging the medium and used as IMR-90 senescent cells (IMR-90_SnSC (Senescent Cells)). Aging of IMR-90 senescent cells was confirmed based on p16, p21 and the activity of senescent-associated beta-galactosidase (SA-β-gal) as indicators.

### Example 2: Immunization of Mouse with IMR-90 Senescent Cell and Acquisition of Monoclonal Antibody

An antibody to a surface antigen of an IMR-90 senescent cell was prepared using lpr/lpr mouse (Japan SLC, Inc., C3H/HeJJms Slc-lpr/lpr). A lpr/lpr mouse was immunized with IMR-90 senescent cells prepared in Example 1 together with an adjuvant (TiterMax Gold, G-1) for inducing an immune reaction. The spleen or lymph node of the immunized mouse was excised in accordance with a routine method. Lymphocytes were collected and subjected to cell fusion with a mouse myeloma cell SP2/0 (ATCC: CRL-1581) to prepare hybridoma cells. Then, a monoclonal antibody was obtained and cultured in a serum-free medium, i.e., CD hybridoma medium (Life Technologies). From the obtained culture supernatant, antibodies were purified using a protein G column (GE HEALTHCARE JAPAN). The obtained monoclonal antibodies were screened by FACS based on binding to IMR-90 senescent cells as an indicator.

As a result, it was found that the antibodies produced from about 20 hybridomas highly reproducibly bind to IMR-90 senescent cells.

### Example 3: Identification of Antigen

For the antibodies confirmed to have binding activity to IMR-90 senescent cells in Example 2, the antigen proteins thereof were attempted to be identified. After the obtained antibodies and a lysate of IMR-90 senescent cells were reacted, antigen proteins were concentrated by immunoprecipitation using protein G sepharose. Using solutions compriseing the antigen proteins bound, amino acid sequences of the antigen proteins were identified by mass spectrometry (MS) according to a routine method.

As a result, from the 20 hybridomas found to bind to IMR-90 senescent cells prepared in Example 1, 15 independent molecular targets were identified. Using a low expression level in a normal tissue and the increased ratio of protein expressions in a non-senescent cell and a senescent cell, as indicators, ITGA11 was selected as an antigen expressed specifically in the senescent cell.

To confirm whether ITGA11 is appropriate as an antigen expressed in the senescent cell, mouse monoclonal antibody A03 (PTL 1) known as an anti-ITGA11 antibody, human monoclonal antibody NB0268 (PTL 2), and chicken-mouse chimeric monoclonal antibody #33-46 (PTL 3) were prepared based on sequence information described in patent publications in accordance with a routine method.

For the antibodies A03, NB0268 and #33-46 mentioned above, the binding activities of them to senescent IMR-90 cells were evaluated by FACS. As a result, it was found that NB0268 and #33-46 antibodies can recognize the senescent cells.

NB0314, described as an antibody improved from NB0268 in physical property in PTL 2, was used for evaluating drug efficacy in mice. A mousenized antibody was prepared by replacing a constant region of human IgG1 with that of mouse IgG2a in accordance with a routine method and designated as anti-ITGA11 antibody "#014". The nucleotide sequence of the heavy chain of anti-ITGA11 antibody #014 prepared is shown in SEQ ID NO: 1 and the amino acid sequence encoded by the nucleotide sequence is shown in SEQ ID NO: 2, the nucleotide sequence of the light chain of the antibody is shown in SEQ ID NO: 3 and the amino acid sequence encoded by the nucleotide sequence is shown in SEQ ID NO: 4.

### Example 4: Evaluation of Senescent-Cell Selective ADCC Activity in vitro

To confirm whether anti-ITGA11 antibody #014 prepared in Example 3 is an antibody inducing ADCC activity by an NK cell in a senescent-cell selective manner, ADCC activity was evaluated by ADCC Reporter Bioassay kit (manufactured by Promega KK., G7010). As a negative control, a human IgG1 antibody (International Publication No. WO2013/094723) to KLH (keyhole limpet hemocyanin), which is an antigen not present *in vivo,* was used.

As a result, the followings are found. The luminescence intensity ratio of anti-ITGA11 antibody #014 in a non-senescent IMR-90 cell is 1.4 relative to that of the cell not comprising the antibody regarded as 1, indicating that antibody #014 rarely induces ADCC activity. However, the ratio was 4.7 in a senescent IMR-90 cell, indicating that antibody #014 induces ADCC activity. The luminescence intensity ratio of an anti-KLH antibody used as a negative control in a non-senescent IMR-90 cell was 1.1 and the ratio thereof in an IMR-90 senescent cell was 1.3.

### Example 5: Evaluation of senescent-cell removal in-vivo

Type 1 (insulin-dependent) diabetes was developed by inducing a damage specific to streptozotocin (STZ)-induced pancreatic β-cell in p16-luc mice (Riken, BRC No. RBRC09236 (Acc. No. CDB0416T-53), male). Based on the suggestion that secondary damage given to organs such as a kidney by long-lasting hyperglycemia is closely connected to cellular senescence (J. Diabetes Complications, Vol. 28 (5), page 604-611 (2014)), since senescent cells in the kidneys of the type 1 diabetes models can be visualized, a treatment effect of the anti-ITGA11 antibody administered was evaluated.

To p16-luc mice (52 weeks old), an STZ solution (200 mg/kg, manufactured by Sigma-Aldrich, S0130, 0.05 mM citric acid-comprising physiological saline) was intraperitoneally administered. Also, to control the degree of pancreatic damage caused by STZ, a nicotinamide solution (100 mg/kg, saline solution) was intraperitoneally administered 90 minutes in advance. Two weeks later, blood (5 µL) was sampled from the tail vein; blood glucose was measured; and the mice were divided into groups.

Two weeks after the administration of STZ, a test substance, i.e., the antibody (anti-ITGA11 antibody #014 (3 mg/kg, 10 mg/kg, or solvent D-PBS (manufactured by FUJIFILM Wako Pure Chemical Corporation, 045-29795), once a week, 3 weeks) was intraperitoneally administered.

Senescent cells were detected by subcutaneously administering 150 mg of luciferin (manufactured by Promega KK., P1043)/10 mL D-PBS/kg solution to p16-luc mice and measuring, 7 minutes later, luminescence from the kidneys under isoflurane anesthesia by IVIS Imaging System (manufactured by PerkinElmer Co., Ltd.). In addition, the amounts of albumin in urine collected for 24 hours up to the day before luciferin administration were measured by ELISA using an anti-albumin antibody (Nordic MUbio, RAM/Alb)

As a result, in the kidneys of STZ-induced diabetes model p16-luc mice, the luminescence of luciferase increased about 10-fold than in the kidneys of mice not receiving STZ. Further, in the kidneys of mice receiving administration of an anti-ITGA11 antibody (10 mg/kg), the luminescence of luciferase was significantly improved by about 50% compared to the amount of luminescence increased by administration of STZ (Fig. 1).

Similarly, the amounts of albumin in the urine of the STZ-induced diabetes model p16-luc mice increased about 21-fold as large as that of mice not receiving STZ. Further, the amounts of albumin in the urine of the mice receiving administration of anti-ITGA11 antibody (10 mg/kg) were significantly improved by about 60% compared to the group receiving administration of STZ (Fig. 2).

From the above results, it was suggested that, in STZ-induced diabetes model mice, the number of senescent cells expressing p16 increases in the kidney resulting in deterioration of the kidney but the kidney function is recovered by removing senescent cells by the anti-ITGA11 antibody.

### INDUSTRIAL APPLICABILITY

It is expected that the pharmaceutical composition of the present invention comprising an anti-ITGA11 antibody is useful for treating or preventing an aging-related disease.

### SEQUENCE LISTING FREE TEXT

SEQ ID NO: 1 represents the nucleotide sequence of the heavy chain of anti-ITGA11 antibody #014.
SEQ ID NO: 2 represents the amino acid sequence of the heavy chain of anti-ITGA11 antibody #014 encoded by SEQ ID NO: 1.
SEQ ID NO: 3 represents the nucleotide sequence of the light chain of anti-ITGA11 antibody #014.
SEQ ID NO: 4 represents the amino acid sequence of the light chain of anti-ITGA11 antibody #014 encoded by SEQ ID NO: 1.

## Claims

1. A pharmaceutical composition for treating or preventing an aging-related disease, wherein the composition comprises an anti-integrin α11 (ITGA11) antibody.

2. The pharmaceutical composition according to claim 1, wherein the anti-ITGA11 antibody is comprised in an effective amount for selectively removing a senescent cell.

3. The pharmaceutical composition according to claim 1 or 2, wherein the senescent cell is a p16-expressing cell.

4. The pharmaceutical composition according to any one of claims 1 to 3, wherein the aging-related disease is selected from the group consisting of:
(i) atherosclerosis, angina pectoris, arrhythmia, cardiomyopathy, congestive heart failure, coronary artery disease, carotid artery disease, endocarditis, coronary artery thrombosis, myocardial infarction, hypertension, aortic aneurysm, diastolic dysfunction of the heart, hypercholesterolemia, hyperlipidemia, mitral valve prolapse, peripheral vascular disease, stress tolerance of the heart, myocardial fibrosis, cerebral aneurysm, and stroke;
(ii) osteoarthritis, osteoporosis, oral mucositis, inflammatory bowel disease, kyphosis, and herniated disc;
(iii) Alzheimer's disease, Parkinson's disease, Huntington's disease, dementia, mild cognitive impairment, and motor neuron dysfunction;
(iv) diabetes, diabetic ulcer, metabolic syndrome, and obesity;
(v) pulmonary fibrosis, chronic obstructive pulmonary disease, asthma, cystic fibrosis, emphysema, bronchiectasis, and decreased pulmonary function;
(vi) macular degeneration, glaucoma, cataract, presbyopia, decreased vision, and loss of vision; and
(vii) kidney disease, kidney failure, diabetic nephropathy, weakness, hearing loss, muscle fatigue, skin disease, skin wound healing, hepatic fibrosis, pancreatic fibrosis, oral submucosal tissue fibrosis, dry eye and sarcopenia;
(viii) residual cancer after chemotherapy, residual cancer after radiotherapy and atrophy or dysfunction of the peripheral normal tissue, dysfunction of tissue transplant associated with ischemia-reperfusion (kidney transplant, heart transplant, liver transplant);
(ix) response failure to vaccine.

5. The pharmaceutical composition according to any one of claims 1 to 4, wherein the ITGA11 antibody comprises a heavy chain consisting of the amino acid sequence represented by SEQ ID NO: 2 and a light chain consisting of the amino acid sequence represented by SEQ ID NO: 4.

6. The pharmaceutical composition according to any one of claims 1 to 5, wherein the ITGA11 antibody comprises a heavy chain consisting of an amino acid sequence encoded by a nucleotide sequence represented by SEQ ID NO: 1 and a light chain consisting of the amino acid sequence encoded by a nucleotide sequence represented by SEQ ID NO: 3.

7. The pharmaceutical composition according to any one of claims 1 to 6, further comprising a pharmaceutically acceptable carrier or additive.

8. A method for treating or preventing an aging-related disease, comprising administering an effective amount of an anti-ITGA11 antibody for selectively removing a senescent cell to a subject.

9. The method according to claim 8, wherein the senescent cell is a p16-expressing cell.

10. The method according to claim 8 or 9, where the aging-related disease is selected from the group consisting of:
(i) atherosclerosis, angina pectoris, arrhythmia, cardiomyopathy, congestive heart failure, coronary artery disease, carotid artery disease, endocarditis, coronary artery thrombosis, myocardial infarction, hypertension, aortic aneurysm, diastolic dysfunction of the heart, hypercholesterolemia, hyperlipidemia, mitral valve prolapse, peripheral vascular disease, stress tolerance of the heart, myocardial fibrosis, cerebral aneurysm, and stroke;
(ii) osteoarthritis, osteoporosis, oral mucositis, inflammatory bowel disease, kyphosis, and herniated disc;
(iii) Alzheimer's disease, Parkinson's disease, Huntington's disease, dementia, mild cognitive impairment, and motor neuron dysfunction;
(iv) diabetes, diabetic ulcer, metabolic syndrome, and obesity;
(v) pulmonary fibrosis, chronic obstructive pulmonary disease, asthma, cystic fibrosis, emphysema, bronchiectasis, and decreased pulmonary function;
(vi) macular degeneration, glaucoma, cataract, presbyopia, decreased vision, and loss of vision; and
(vii) kidney disease, kidney failure, diabetic nephropathy, weakness, hearing loss, muscle fatigue, skin disease, skin wound healing, hepatic fibrosis, pancreatic fibrosis, oral submucosal tissue fibrosis, dry eye and sarcopenia;
(viii) residual cancer after chemotherapy, residual cancer after radiotherapy and atrophy or dysfunction of the peripheral normal tissue, dysfunction of tissue transplant associated with ischemia-reperfusion (kidney transplant, heart transplant, liver transplant);
(ix) response failure to vaccine.

11. The method according to any one of claims 8 to 10, wherein the ITGA11 antibody comprises a heavy chain consisting of an amino acid sequence represented by SEQ ID NO: 2 and a light chain consisting of an amino acid sequence represented by SEQ ID NO: 4.

12. The method according to any one of claims 8 to 11, wherein the ITGA11 antibody comprises a heavy chain consisting of an amino acid sequence encoded by a nucleotide sequence represented by SEQ ID NO: 1 and a light chain consisting of an amino acid sequence encoded by the nucleotide sequence represented by SEQ ID NO: 3.

13. An anti-ITGA11 antibody for use in treating or preventing an aging-related disease.

14. Use of an anti-ITGA11 antibody for treating or preventing an aging-related disease.

15. Use of an anti-ITGA11 antibody for producing a pharmaceutical composition for treating or preventing an aging-related disease.
